# EUROPEAN PATENT SPECIFICATION

(11) **EP 1 436 621 B1**
(45) Date of publication and mention of the grant of the patent: **22.12.2010**
(21) Application number: 02798889.8
(22) Date of filing: 17.09.2002
(51) Int. Cl.: G01N 33/543, C12Q 1/68

(54) **MULTI-ANALYTE ASSAY DEVICE WITH MULTI-SPOT DETECTION ZONE**
MEHRFACHANALYTASSAY-VORRICHTUNG MIT MULTI-SPOT-NACHWEISZONE
DISPOSITIF DE TEST MULTI-ANALYTES A ZONE DE DETECTION MULTIPOINTS

(30) Priority: 17.09.2001 SE 0103072; 17.09.2001 US 322616 P
(43) Date of publication of application: 14.07.2004
(73) Proprietor: Phadia AB, 751 37 Uppsala (SE)
(72) Inventor: MENDEL-HARTVIG, Ib, S-756 55 Uppsala (SE); BJÖRKMAN, Rune, S-752 36 Uppsala (SE); RUNDSTRÖM, Gerd, S-752 41 Uppsala (SE)
(74) Representative: Lindgren, Anders
(86) International application number: PCT/SE2002/001671
(87) International publication number: WO 2003/025573

(56) References cited:
- EP-A2- 1 018 649
- WO-A1-99/36777
- US-A- 4 591 570
- US-A- 5 244 815
- US-A- 5 858 732
- US-A- 6 100 099

## Description

### Field of the invention

The present invention relates to a solid phase assay device comprising a multi-spot detection zone, and to use thereof in immunochromatographic assays.

### Background of the invention

A type of solid phase assay devices comprises a plate-shaped flow matrix of bibulous material, usually a membrane strip, such as of cellulose nitrate or glass fibre, in which liquid can be transported laterally (i.e. in the plane of the strip) by capillary forces in the membrane. The membrane usually has a sample application zone and a detection zone downstream of the sample application zone. In the detection zone, usually a capturing reagent for the analyte is immobilised. To conduct an assay, the application zone is contacted with the liquid sample to be assayed for the analyte of interest. The device is maintained under conditions sufficient to allow capillary action of liquid to transport the analyte of interest, if present in the sample, through the membrane strip to the detection zone where the analyte is captured. An absorbing pad or the like at the downstream end of the strip usually insures the capillary liquid flow. A detection reagent, usually labelled, is then added upstream of the detection zone and interacts with captured analyte in the detection zone, and the amount of captured analyte is measured. Often, the detection reagent is pre-disposed in or on the membrane strip, e.g. in the form of diffusely movable particles containing fluorophoric or chromogenic groups, either upstream of the sample application zone or between sample application zone and the detection zone.

A major drawback with these known devices is that only a few analytes can be measured per assay.

In EP 191 640 (Syntex Inc) there is disclosed a device in which more than one analyte may be detected. However, the number of analytes that may be detected is limited and the problem of detecting cross-reacting analytes is not addressed.

### Summary of the invention

The problem underlying the present invention was to enable detection of several analytes and even analytes which cross-react with each other, such as different allergens reacting with the same IgE's.

This problem has been solved by a multi-spot device according to the present invention.

Thus, in a first aspect the invention provides a device for determining analytes in an aqueous sample comprising:
an elongate flow matrix being a porous membrane allowing lateral transport of fluid therethrough, wherein said matrix comprises a sample application zone and downstream thereof, a detection zone having immobilised capture agents capable of directly or indirectly binding to said analytes, wherein said analytes are detected by allowing a labelled second binding agent to bind directly or indirectly to the analytes, wherein A) the immobilised capture agents are distributed in the detection zone as a plurality of small spots, thereby permitting multi-analyte and/or multi-specificity detection, and B) the capture agents are anchored to the matrix via immobilised particles, and C) the number of spots per flow matrix is more than 100, and D) wherein some of the spots functions as positive control(s) and/or internal calibrator(s) wherein spots comprising capture agents having the capability to cross-react with analytes are arranged in different flow lines of liquid, and the capture agents are allergens or autoantigens or immunoactive fragments thereof.

The number of spots per flow matrix is preferably 100-1000. The spots are preferably smaller than 1 mm in diameter, preferably smaller than 0.5 mm in diameter.

The spots are preferably arranged in a pattern that allows for detection of cross reactive analytes or specificities. This is exemplified by allergens having cross-reacting IgE, i.e. such allergens should not be arranged in the same flow line.

The flow matrix is a porous membrane, such as nitro-cellulose or a strip of solid material.

The capture agents are allergens or auto-antigens or an immunoactive fragment thereof.

In the device some of the spots functions as positive control(s) and/or internal calibrator(s).

The sample is whole blood, serum, plasma, saliva or urine.

The label of the labelled second binding reagent is, for example a fluorophore or a chromophore.

The device may be used for screening of unknown analytes as well as for detection of specific immunoglobulins. By depositing many spots with known material, for example protein, it is possible to rapidly screen for which binder(s) there are in a sample that are specifically binding to the material in particular spot(s). An example is sample determination of specific IgE, wherein the spots contain different allergens.

### Brief description of drawings

Fig. 1 is a perspective view of an embodiment of a device according to the present invention.
Fig. 2 is a sectional side view of the device in Fig 1;
Fig. 3 is an exploded view corresponding to the side view in Fig. 2.

### Detailed description of the invention

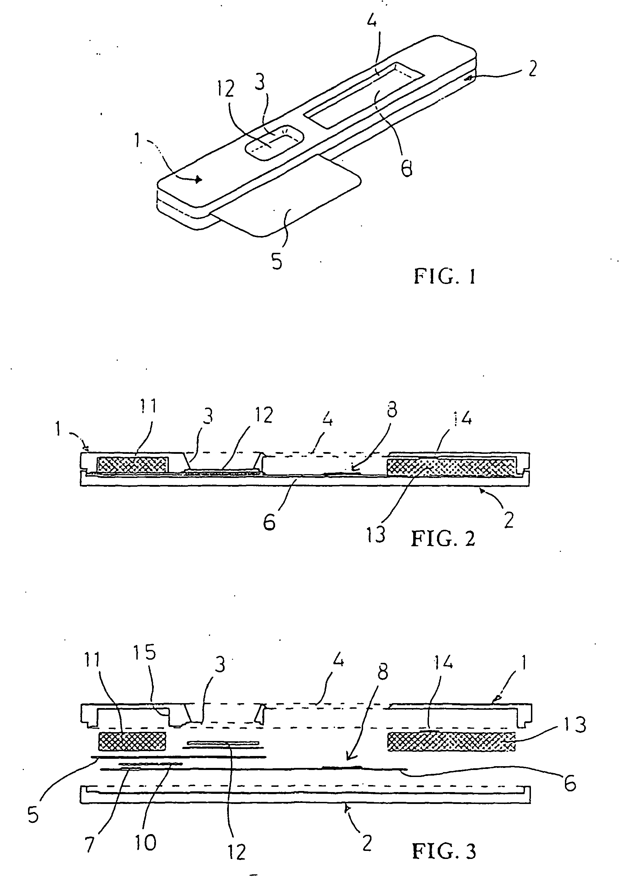

As shown in Fig. 1 the device comprises an upper housing part 1 and a lower housing part 2 of material which is inert with respect to the sample an any reagents used in the assays to be conducted with the device, e.g. polystyrene or polypropylene. The upper housing part 1 has a sample well aperture 3 (here conical) and a detection window 4.

The lower housing part 2 has mounted therein a membrane strip 6 of biboulous material (i.e. a porous material susceptible to traversal of an aqueous medium due to capillary action), e.g. nitro-cellulose on a polyester backing. Near the upstream end of the strip 6 (to the left in the figures), a filter piece 7, containing diffusely movable detection reagent (labelled second binding reagent), is placed on the strip. Such a detection reagent may, for example, be a conjugate between a label particle and a reactant capable of binding to the analyte. Further downstream, and placed below and within the detection window 4, there is a multi-spot reaction zone 8 on the strip which contains several capturing agents or reactants immobilised in a specific pattern on the strip. The capturing agents are capable of binding to the analytes to be tested for. The reaction zone 8 (Fig. 2-3) may be smaller or larger than in the shown figures and may contain 100-1000 capturing agents. Importantly, capturing agents having cross-reacting analytes will not be arranged in the same lane, i.e. not in the same flow line of liquid.

The upper housing part 1 contains at the upstream end of the membrane strip 6, a pad 11 of liquid absorbing material intended to serve as a container for flow liquid, or buffer. The opening 3 in the housing part 1 is intended for introducing sample to the membrane 6. In the illustrated case, a filter element 12 (which optionally may consist of two or more filters), is provided below the opening 3 for assays where the sample liquid needs to be filtered, e.g. when the sample is whole blood and blood cells are to be separated off. The buffer pad 11 thus forms a buffer liquid container, below referred to as buffer pad, and the room defined by the sample opening 3 and the filter element 12 forms a sample well, or sample container.

Optionally, a pull-out film 5 is present the purpose of which will be described further below. At the downstream end of the membrane strip 6, a wicking element 13 is placed, here in the form of a pad of absorbent material, such as cellulose, the purpose of which is to assist in maintaining a capillary flow of assay liquids through the membrane strip 6.

An assay for analytes in a sample may be performed with the device described above as follows.

The device is usually provided ready for use with the buffer pad 11 soaked with buffer solution (flow liquid), with the detection reagent pre-deposited in the filter 7, and with the respective appropriate capture agents and calibration agents immobilised in a specific pattern of spots in the reaction (or detection) zone 8. This offers a possibility to optimally position the calibration spots among the other spots.

The function of the calibration spots is as a positive control and/or internal calibrator.

A predetermined amount of sample is added through the opening 3 in the housing part 1. All the necessary assay liquids, i.e. in this case sample liquid and buffer liquid, are then present in the device, the pull-out film 5, however, effectively preventing contact between the respective liquids and the membrane strip 6. The assay is then started by the operator removing the pull-out film 5 to thereby put the membrane strip 6 in simultaneous liquid receiving contact with the buffer pad 11 and the sample liquid in the sample well 3. If the pull-out film is not present, the assay will start directly following sample addition.

Buffer liquid from the pad 11 will now penetrate into the membrane strip 6 via the far upstream end part thereof which is in direct contact with the pad 11 (see Fig. 3) and be transported downstream the membrane strip 6 by capillary force. Simultaneously, sample liquid directly followed by a (first) flow pulse of buffer liquid. However, the detection reagent filter 7 and a major part of the buffer pad 11 are separated from the membrane strip 6 by the flow barrier film 10. Buffer liquid that has been transported into the membrane strip 6 will penetrate into and be transported through the filter 7 and bring the detection reagent deposited therein with it, thereby forming a detection reagent flow pulse. This detection reagent flow pulse will follow in sequence after the sample flow and the buffer flow pulse. Buffer that is transported in the membrane strip 6 after the detection reagent has been removed from the filter 7 will form a second buffer flow pulse following after the detection reagent flow pulse.

The above-mentioned different liquid flows will be transported along the membrane strip 6 in the indicated sequence, i.e. sample flow, first buffer flow, detection reagent flow, and second buffer flow, and will eventually reach the multi-spot reaction zone 8. In the reaction zone 8, analytes present in the sample will be captured by the reagents immobilised in the specific spot pattern in the membrane. The analyte/capture reagent complexes formed will be washed by the following first buffer flow, and the flow of detection reagent will form detectable reagent/analyte complexes in the reaction zone. The latter will finally be washed by the second buffer flow. In the calibration spots, the predetermined amount of analyte therein will react with the detection reagent in the detection reagent flow to form a detectable detection reagent/analyte complex. By measuring the signal intensity from the detection reagent captured in the reaction zone and correlate it with that obtained in the calibration spot(s), the amount of analyte in the sample may be determined.

In the reaction (or detection) zone 8 described above, several reactants capable of specifically binding to analytes are immobilised in a specific spot pattern (by covalent binding, via physical adsorption, via biospecific affinity, via immobilised particles to which the reactant is covalently bound, etc.). However, instead an agent capable of reacting with the reactant may be immobilised in the membrane, and the reactant may then be added together with the sample, or be pre-deposited in the membrane in an area or zone upstream of the reaction zone. Such an immobilised agent may be one member of a specific binding pair (sbp) and the reactant is then coupled or conjugated to the other member of the spb.

Similarly, the calibration spot(s) may contain a binder for the calibrator substance rather than the calibrator substance *per se*. The binder is usually a member of a specific binding pair, such as one of those mentioned above, whereas the other member of the specific binding pair is coupled or conjugated to the calibrator substance, which may in turn be added with the sample or pre-deposited upstream of the calibrator zone. Streptavidin, for example, may be immobilised in the calibrator zone while the calibrator substance is biotinylated.

For further details on assay devices of the type contemplated herein, and particularly regarding flow matrixes, sequential assays, calibrator systems and detection reagents, it may be referred to our published PCT applications WO 99/36776, WO 99/36777 and WO 99/36780, for example.

Analytes to be determined using the present device are readily apparent to the skilled person. The analyte is an antibody.

The present device permits convenient pre-treatment of the sample before starting the assay.

The present device may also be adapted for performing assays of the type described in our published PCT application WO 99/60402 where the flow matrix contains a chromatographic separation zone upstream of the reaction (detection) zone to separate sample components which would otherwise disturb or influence the determination of the analyte.

## Claims

1. A device for determining analytes in an aqueous sample comprising: an elongate flow matrix (6), being a porous membrane, allowing lateral transport of fluid therethrough, wherein said matrix comprises a sample application zone (3) and downstream thereof, a detection zone (8) having immobilised capture agents capable of directly or indirectly binding to said analytes, wherein said analytes are detected by allowing a labelled second binding agent to bind directly or indirectly to the analytes, and
the immobilised capture agents are distributed in the detection zone (8) as a plurality of small spots, thereby permitting multi-analyte and/or multi-specificity detection,
the capture agents are anchored to the matrix via immobilised particles, and
some of the spots functions as positive control(s) and/or internal calibrator(s),
**characterised in that**
A) the number of spots per flow matrix is more than 100
B) spots comprising capture agents having the capability to cross-react with analytes are arranged in different flow lines of liquid, and
C) the capture agents are allergens or autoantigens or immunoactive fragments thereof.

2. A device according to claim 1, wherein the spots are smaller than 1 mm in diameter, preferably smaller than 0,5 mm in diameter.

3. A device according to any one of the above claims, wherein the sample is whole blood, serum, plasma, saliva or urine.

4. A device according to any of the above claims, wherein the label is a fluorophore or a chromophore.

5. Use of the device according to one or more of the above claims 1-4 for screening of unknown analytes.

6. Use of the device according to one or more of the above claims 1-4 for screening of specific immunoglobulins.

## Patentansprüche

1. Vorrichtung zur Bestimmung von Analyten in einer wässrigen Probe umfassend:
eine längliche Durchflussmatrix (6), die eine poröse Membran ist und lateralen Transport von Flüssigkeit durch sie erlaubt, wobei die Matrix eine Probenauftragszone (3) und stromabwärts davon eine Nachweiszone (8) umfasst, die immobilisierte Einfangmittel aufweist, die direkt oder indirekt an die Analyten binden können, wobei die Analyten **dadurch** nachgewiesen werden,
dass einem markierten zweiten Bindemittel erlaubt wird, direkt oder indirekt an die Analyten zu binden, und
die immobilisierten Bindemittel in der Nachweiszone (8) als eine Vielzahl von kleinen Spots verteilt sind, wodurch Multi-Analyt- und/oder Multi-Spezifitäten-Nachweis erlaubt wird;
die Bindemittel in der Matrix über immobilisierte Partikel verankert sind, und einige der Spots als Positivkontrolle(n) und/oder interne Eichvorrichtung(en) fungieren, **dadurch gekennzeichnet, dass**
A) die Anzahl der Spots pro Durchflussmatrix größer als 100 ist,
B) die Spots, die Bindemittel umfassen, die die Fähigkeit haben, mit Analyten kreuzzureagieren, in verschiedenen Flüssigkeitsdurchflusslinien angeordnet sind, und
C) die Bindemittel Allergene oder Autoantigene oder immunreaktive Fragmente davon sind.

2. Vorrichtung nach Anspruch 1, wobei die Spots kleiner als 1 mm im Durchmesser, bevorzugt kleiner als 0,5 mm im Durchmesser, sind.

3. Vorrichtung nach einem der vorherigen Ansprüche, wobei die Probe Gesamtblut, Serum, Plasma, Speichel oder Urin ist.

4. Vorrichtung nach einem der vorherigen Ansprüche, wobei die Markierung ein Fluorophor oder ein Chromophor ist.

5. Verwendung der Vorrichtung nach einem oder mehreren der vorherigen Ansprüche 1 bis 4 zum Screenen nach unbekannten Analyten.

6. Verwendung der Vorrichtung nach einem oder mehreren der vorherigen Ansprüche 1 bis 4 zum Screenen nach spezifischen Immunglobulinen.

## Revendications

1. Dispositif pour déterminer des analytes dans un échantillon aqueux comprenant : une matrice de flux allongé (6), qui est une membrane poreuse, permettant un transport latéral de fluide à travers celle-ci, dans lequel ladite matrice comprend une zone d'application d'échantillon (3) et en aval de celle-ci, une zone de détection (8) sur laquelle sont immobilisés des agents de capture capables de se lier directement ou indirectement auxdits analytes, dans lequel lesdits analytes sont détectés en laissant un second agent de liaison marqué se lier directement ou indirectement aux analytes, et
les agents de capture immobilisés sont répartis dans la zone de détection (8) sous la forme d'une pluralité de petits points, permettant ainsi une détection multi-analyte et/ou multi-spécificité,
les agents de capture sont ancrés dans la matrice via des particules immobilisées, et
certains des points fonctionnent comme un ou plusieurs témoins positifs et/ou calibreurs internes,
**caractérisé en ce que**
A) le nombre de points par matrice de flux est supérieur à 100,
B) les points comprenant des agents de capture ayant la capacité d'inter-réagir avec des analytes sont agencés dans différentes rangées d'écoulement de liquide, et
C) les agents de capture sont des allergènes ou des auto-antigènes ou leurs fragments immunoactifs.

2. Dispositif selon la revendication 1, dans lequel les points ont un diamètre inférieur à 1 mm, de préférence inférieur à 0,5 mm.

3. Dispositif selon l'une quelconque des revendications précédentes, dans lequel l'échantillon est constitué de sang complet, de sérum, de plasma, de salive ou d'urine.

4. Dispositif selon l'une quelconque des revendications précédentes, dans lequel le marqueur est un fluorophore ou un chromophore.

5. Utilisation du dispositif selon une ou plusieurs des revendications 1 à 4 précédentes, pour dépister des analytes inconnus.

6. Utilisation du dispositif selon une ou plusieurs des revendications 1 à 4 précédentes pour dépister des immunoglobulines spécifiques.
